(19) Europäisches Patentamt
European Patent Office
Office européen des brevets

(11) **EP 3 656 294 A1**

(12) **EUROPEAN PATENT APPLICATION**

(43) Date of publication:
**27.05.2020 Bulletin 2020/22**

(51) Int Cl.:
***A61B 5/0215*** (2006.01)

(21) Application number: **18207789.1**

(22) Date of filing: **22.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DIRKSEN, Peter**
**5656 AE Eindhoven (NL)**

• **MAUCZOK, Ruediger Günther**
**5656 AE Eindhoven (NL)**
• **DRENTH, Henk**
**5656 AE Eindhoven (NL)**
• **AGRICOLA, Franciscus Theodorus**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CAPACITIVE PRESSURE SENSOR FOR INTRALUMINAL GUIDEWIRE OR CATHETER**

(57) A capacitive pressure sensor for a pressure-sensing guidewire or catheter includes a substrate; a first active cell formed in the substrate; a second active cell formed in the substrate, wherein the first active cell and the second active cell are electrically active to generate electrical signals representative of an external pressure; and a dummy cell formed in the substrate, wherein the dummy cell is electrically inactive such that no electrical signal representative of the external pressure is generated. An intraluminal pressure-sensing device and a system are also provided.

Fig. 3

**Description**

TECHNICAL FIELD OF THE INVENTION

The present disclosure relates to capacitive pressure sensors for intraluminal pressure-sensing guidewires and/or catheters. In some embodiments, a capacitive pressure sensor with three capacitive cells is provided.

BACKGROUND OF THE INVENTION

Heart disease is very serious and often requires emergency operations to save lives. A main cause of heart disease is the accumulation of plaque inside the blood vessels, which eventually occludes the blood vessels. Common treatment options available to open up the occluded vessel include balloon angioplasty, rotational atherectomy, and intravascular stents. Traditionally, surgeons have relied on X-ray fluoroscopic images that are planar images showing the external shape of the silhouette of the lumen of blood vessels to guide treatment. Unfortunately, with X-ray fluoroscopic images, there is a great deal of uncertainty about the exact extent and orientation of the stenosis responsible for the occlusion, making it difficult to find the exact location of the stenosis. In addition, though it is known that restenosis can occur at the same place, it is difficult to check the condition inside the vessels after surgery with X-ray.

A currently accepted technique for assessing the severity of a stenosis in a blood vessel, including ischemia causing lesions, is fractional flow reserve (FFR). FFR is a calculation of the ratio of a distal pressure measurement (taken on the distal side of the stenosis) relative to a proximal pressure measurement (taken on the proximal side of the stenosis). FFR provides an index of stenosis severity that allows determination as to whether the blockage limits blood flow within the vessel to an extent that treatment is required. The normal value of FFR in a healthy vessel is 1.00, while values less than about 0.80 are generally deemed significant and require treatment.

Another way of assessing blood vessels is to use ultrasound imaging. For example, ultrasound imaging arrays formed of capacitive micromachined ultrasound transducers (CMUTs) have been investigated. Manufacturing CMUT arrays, however, is difficult because of non-uniformities that arise. For example, membranes behave differently based on whether they are located in the middle of the array or whether they are at the edge or corner of the array. Such non-uniformity is untenable because medical sensors need to behave in the same, predictable manner. These challenges prevent the full range of capacitive sensors for assessing blood vessels from being realized.

SUMMARY OF THE INVENTION

Embodiments of the present disclosure are directed to capacitive pressure sensors for intraluminal guidewires and/or catheters. For example, the capacitive pressure sensor can be implemented in an intravascular guidewire and used to measure the pressure of blood flow within the blood vessel of a patient. The capacitive pressure sensor includes two active cells and one dummy cell. The two active cells electrically communicate with other components of the capacitive pressure sensor and are used to measure pressure. The dummy cell does not communicate with other components and is not used to measure pressure. The dummy cell is located on the capacitive pressure sensor because of an advantageous fabrication process that ensures that the active cells behave in the same, expected way for each capacitive pressure sensor. In that regard, the silicon wafer on which the capacitive pressure sensors are fabricated include a spatially compact distribution of capacitive cells, with many dummy cells surrounding the active cells. This dense arrangement advantageously ensures that the active cells do not have any processing non-uniformities that adversely affect how the active cells measure pressure.

In an exemplary aspect, a capacitive pressure sensor for a pressure-sensing guidewire or catheter is provided. The capacitive pressure sensor includes a substrate; a first active cell formed in the substrate; a second active cell formed in the substrate, wherein the first active cell and the second active cell are electrically active to generate electrical signals representative of an external pressure; and a dummy cell formed in the substrate, wherein the dummy cell is electrically inactive such that no electrical signal representative of the external pressure is generated.

In one aspect, the first active cell and the second active cell are spaced from one another by a first pitch. In one aspect, the dummy cell is spaced from at least one of the first active cell or the second active cell by a different, second pitch. In one aspect, the first active cell, the second active cell, and the dummy cell are arranged longitudinally along the substrate. In one aspect, the first active cell and the second active cell comprise a first diameter, and the dummy cell comprises a different, second diameter. In one aspect, the capacitive pressure sensor further includes an integrated circuit disposed in the substrate, wherein the integrated circuit is in communication with the first active cell and the second active cell. In one aspect, the capacitive pressure sensor further includes a first bond pad formed in the substrate; and a second bond pad formed in the substrate, wherein the first bond pad and the second bond pad are in communication with the integrated circuit. In one aspect, the first active cell and the second active cell are symmetrical about an axis of the substrate. In one aspect, the substrate comprises a proximal portion comprising a first dimension and a distal portion comprising a smaller, second dimension, and the first active cell and second active cell are formed in the distal portion. In one aspect, the capacitive pressure sensor further includes an integrated circuit disposed in the distal portion of the substrate, wherein the integrated circuit is in communication with the first active cell and the second active cell.

In an exemplary aspect, an intraluminal pressure-sensing device is provided. The intraluminal pressure-sensing device includes a flexible elongate member configured to be positioned within a body lumen of a patient; a capacitive pressure sensor coupled to the flexible elongate member, wherein the capacitive pressure sensor comprises: a substrate; a first active cell formed in the substrate; a second active cell formed in the substrate, wherein the first active cell and the second active cell are electrically active to generate electrical signals representative of an external pressure; and a dummy cell formed in the substrate, wherein the dummy cell is electrically inactive such that no electrical signal representative of the external pressure is generated.

In one aspect, the intraluminal pressure-sensing device further includes an integrated circuit disposed in the substrate, wherein the integrated circuit is in communication with the first active cell and the second active cell, wherein the integrated circuit is configured to output an electrical signal representative of a sensed pressure at the first active cell and the second active cell. In one aspect, the intraluminal pressure-sensing device further includes a first electrical conductor and a second electrical conductor only, wherein the first electrical conductor and the second electrical conductor are in communication with the integrated circuit, wherein the capacitive pressure sensor is coupled to a distal portion of the flexible elongate member, wherein the first electrical conductor and the second electrical conductor extend from the distal portion of the flexible elongate member to a proximal portion of the flexible elongate member, and wherein at least one of the first electrical conductor or the second electrical conductor are configured to transmit the electrical signal representative of the sensed pressure from the capacitive pressure sensor at the distal portion of the flexible elongate member to a connector at the proximal portion of the flexible elongate member. In one aspect, the intraluminal pressure-sensing device further includes a first bond pad formed in the substrate; and a second bond pad formed in the substrate, wherein the first bond pad and the second bond pad are in communication with the integrated circuit, and wherein the first electrical conductor and the second electrical conductor are respectively in communication with the first bond pad and the second bond pad. In one aspect, the intraluminal pressure-sensing device further includes a housing coupled to the flexible elongate member, wherein the capacitive pressure sensor is disposed within the housing. In one aspect, the substrate of the capacitive pressure sensor comprises a proximal portion coupled to the housing and a cantilevered distal portion, wherein the first active cell and the second active cell are formed in the cantilevered distal portion. In one aspect, the intraluminal pressure-sensing device further includes an integrated circuit disposed in the cantilevered distal portion of the substrate, wherein the integrated circuit is in communication with the first active cell and the second active cell. In one aspect, the flexible elongate member comprises a guidewire. In one aspect, the flexible elongate member comprises a catheter.

In an exemplary aspect, a system is provided. The system includes a pressure-sensing catheter or guidewire comprising a capacitive pressure sensor, wherein the capacitive pressure sensor comprises: a substrate; a first active cell formed in the substrate; a second active cell formed in the substrate, wherein the first active cell and the second active cell are electrically active to generate electrical signals representative of an external pressure; and a dummy cell formed in the substrate, wherein the dummy cell is electrically inactive such that no electrical signal representative of the external pressure is generated; and a computer in communication with the capacitive pressure sensor, wherein the computer is configured to generate a pressure value based on the electrical signals representative of the external pressure and to output, to a display, a visual representation of the pressure value.

Additional aspects, features, and advantages of the present disclosure will become apparent from the following detailed description.

BRIEF DESCRIPTION OF THE DRAWINGS

Illustrative embodiments of the present disclosure will be described with reference to the accompanying drawings, of which:

Fig. 1 is a diagrammatic schematic view of an intraluminal pressure-sensing system, according to an embodiment of the present disclosure.
Fig. 2 is a diagrammatic top view of an intraluminal pressure-sensing device, according to an embodiment of the present disclosure.
Fig. 3 is a diagrammatic perspective view of a capacitive pressure sensor, according to an embodiment of the present disclosure.
Fig. 4 is a diagrammatic top view of a capacitive pressure sensor disposed within a housing of an intraluminal device, according to an embodiment of the present disclosure.
Fig. 5 is a diagrammatic cross-sectional view of the capacitive pressure sensor of Fig. 3 along section line 5-5, according to an embodiment of the present disclosure.
Fig. 6 is a diagrammatic cross-sectional view of a distal portion of an intraluminal device including the capacitive pressure sensor of Fig. 4 along section line 6-6, according to an embodiment of the present disclosure.
Fig. 7 is a diagrammatic cross-sectional view of a distal portion of an intraluminal device including the capacitive pressure sensor of Fig. 6 along section line 7-7, according to an embodiment of the present disclosure.

Fig. 8 is a diagrammatic top view of a wafer during fabrication of the capacitive pressure sensor, according to another embodiment of the present disclosure.

Fig. 9 is a diagrammatic top view of a die during fabrication of a capacitive pressure sensor, according to another embodiment of the present disclosure.

Fig. 10 is a diagrammatic top view of a region of the die of Fig. 9, according to another embodiment of the present disclosure.

Fig. 11 is a diagrammatic top view of a region of the die of Fig. 10, according to another embodiment of the present disclosure.

DETAILED DESCRIPTION OF THE EMBODIMENTS

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

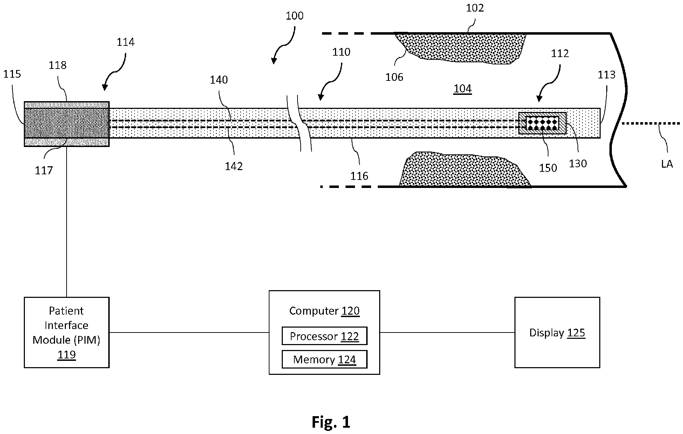

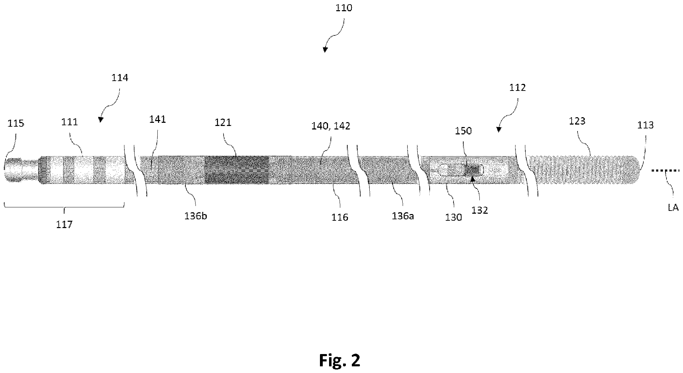

Referring now to Figs. 1 and 2, shown therein are one or more components of a system 100, including an intraluminal device 110, according to embodiments of the present disclosure. Fig. 1 is a diagrammatic schematic view of the system 100. Fig. 2 is a diagrammatic top view of the intraluminal device 110. The system 100 includes the intraluminal device 110, a patient interface module (PIM) 119, a computer 120, and a display 125. The system 100 can also be referenced as an intraluminal system, a pressure-sensing system, a diagnostic system, and/or combinations thereof. In general, the intraluminal device 110 obtains data associated with the anatomy 102 while positioned within the anatomy 102. In some instances, the intraluminal device 110 obtains data representative of the pressure of fluid flow within a lumen 104 of the anatomy 102 while the intraluminal device 110 is positioned within the lumen 104. For example, the intraluminal device 110 obtains pressure data of blood flow within a blood vessel. The computer 120 receives the pressure data via the PIM 119, processes the pressure data, and generates a visual representation of the pressure data that is displayed by the display 125.

The intraluminal device 110 can be a guidewire, catheter, and/or guide catheter. The intraluminal device 110 can be referenced as a pressure-sensing device, a pressure-sensing guidewire, a pressure-sensing catheter, a diagnostic device, and/or combinations thereof in some instances. In the illustrated embodiment of Fig. 2, the intraluminal device 110 is a guidewire. The intraluminal device 110 includes a flexible elongate member 116 and a pressure sensor 150 coupled thereto. The flexible elongate member 116 may be a thin, long, flexible structure sized and shaped, structurally arranged, and/or otherwise configured to be positioned within the lumen 104 of the anatomy 102. The flexible elongate member includes a distal portion 112 adjacent a distal end 113 and a proximal portion 114 adjacent a proximal end 115. In some instances, the distal portion includes any portion of the flexible elongate member 116 from the mid-point to the distal end 113, and the proximal portion includes any portion of the flexible elongate member 116 from the mid-point to the proximal end 115. A middle portion is disposed between the distal portion 112 and the proximal portion 114. During use, the distal portion 112 and a majority of the middle portion is positioned within the lumen 104 of the anatomy 102 while the proximal portion 114 is positioned outside of the body of the patient. The flexible elongate member 116 can include a longitudinal axis LA. In some instances, the longitudinal axis LA can be a central longitudinal axis of the flexible elongate member 116. All or a portion of the flexible elongate member 116 may have any suitable geometric cross-sectional profile (*e.g.*, circular, oval, rectangular, square, elliptical, etc.) or non-geometric cross-sectional profile. For example, the flexible elongate member 116 can have a generally cylindrical profile with a circular cross-sectional profile that defines an outer diameter of the flexible elongate member 116. For example, the outer diameter of the flexible elongate member 116 can be any suitable value for positioning within the body lumen of the patient, including between approximately 0.5 Fr and approximately 15 Fr, including values such as 0.5 Fr, 1 Fr, 1.05 Fr, 1.10 Fr, 1.5 Fr, 2 Fr, 3.5 Fr, 5 Fr, 7 Fr, 8.2 Fr, 9 Fr, and/or other suitable values both larger and smaller. The components forming the intraluminal device 110 are sized and shaped, structurally arranged, and/or otherwise configured to allow for the diameter of the flexible elongate member 116 to be very small. For example, the outside diameter of the flexible elongate member 116 can be between about 0.0007" (0.0178 mm) and about 0.118" (3.0 mm), with some particular embodiments having outer diameters of approximately 0.014" (0.3556 mm) and approximately 0.018" (0.4572 mm)). While the total length of the flexible elongate member 116 can be any length, in some embodiments the total length is between about 1300 mm and about 4000 mm, with some specific embodiments have a length of 1400 mm, 1750 mm, 1850 mm, 1900 mm, and 3000 mm.

The flexible elongate member 116 can be tubular in shape in some instances. The intraluminal device 110 may or may not include one or more lumens extending along all or a portion of the length of the flexible elongate member 116. One or more components forming the intraluminal device 110 can be positioned within the one or more lumens of the flexible elongate member 116. The lumen of the flexible elongate member 116 can be sized and shaped, structurally arranged, and/or otherwise configured to receive and/or guide one or more other diagnostic and/or therapeutic instruments. If the flexible elongate member 116 includes lumen(s), the lumen(s) may be centered or offset with respect to the cross-sectional profile of the intraluminal device 110. The intraluminal device 110 can be a catheter including a lumen configured to receive a guidewire. During a diagnostic and/or therapeutic procedure, a medical professional typically first inserts the guidewire into the body lumen and moves the guidewire to a desired location within the anatomy. The guidewire facilitates introduction and positioning of one or more other diagnostic and/or therapeutic instruments, such as a catheter, at the desired location within the anatomy. In some embodiments, the lumen of the intraluminal device 110 can extend along the entire length or a portion of the length of the flexible elongate member 116.

The flexible elongate member 116 can include any suitable components formed of different materials. As shown Fig. 2, the flexible elongate member 116 includes core wires 136a, 136b, which provide internal structure for the intraluminal device 110. The core wires 136a, 136b can be formed of a metal or metal alloy in various embodiments. The diameter of the cores wires 136a, 136b can be the same or vary along the length of the flexible elongate member. The core wires 136a, 136b can be coupled by adhesive, solder, and/or other attachment mechanism within a hypotube 121, which also forms part of the flexible elongate member. The core wire 136a extends along the distal portion 112 and/or a middle portion of the flexible elongate member 116, while the core wire 136b extends along the proximal portion. In other embodiments, the intraluminal device includes only one core wire. The flexible elongate member 116 can include one or more polymer or plastic layers and/or coatings surrounding the core wires 136a, 136b. The distal portion of the flexible elongate member 116 includes a coil 123 and terminates in a solder ball at the distal end 113.

The anatomy 102 may represent any fluid-filled or surrounded structures, both natural and man-made. For example, the anatomy 102 can be within the body of a patient. Fluid can flow through the lumen 104 of the anatomy 102. The lumen 104 can be referenced as a body lumen in some instances. The anatomy 102 can be a vessel, such as a blood vessel, in which blood flows through the lumen 104. In such instances, the intraluminal device 110 can be referenced as an intravascular device. In various embodiments, the blood vessel is an artery or a vein of a patient's vascular system, including cardiac vasculature, peripheral vasculature, neural vasculature, renal vasculature, and/or any other suitable anatomy/lumen inside the body. The anatomy 102 can be tortuous in some instances. For example, the intraluminal device 110 may be used to examine any number of anatomical locations and tissue types, including without limitation, organs including the liver, heart, kidneys, gall bladder, pancreas, lungs, esophagus; ducts; intestines; nervous system structures including the brain, dural sac, spinal cord and peripheral nerves; the urinary tract; as well as valves within the blood, chambers or other parts of the heart, and/or other systems of the body. In addition to natural structures, the intraluminal device 110 may be used to examine man-made structures such as, but without limitation, heart valves, stents, shunts, filters and other devices. Embodiments of the present disclosure are particularly suited for use in the context of human anatomy. In some aspects, the present disclosure can be generally used in a lumen of an anatomical or non-anatomical structure, including both medical and non-medical applications.

The occlusion 106 of the anatomy 102 is generally representative of any blockage or other structural arrangement that results in a restriction to the flow of fluid through the lumen 104, for example, in a manner that is deleterious to the health of the patient. For example, the occlusion 106 narrows the lumen 104 such that the cross-sectional area of the lumen 104 and/or the available space for fluid to flow through the lumen 104 is decreased. Where the anatomy 102 is a blood vessel, the occlusion 106 may be a result of plaque buildup, including without limitation plaque components such as fibrous, fibro-lipidic (fibro fatty), necrotic core, calcified (dense calcium), blood, fresh thrombus, and/or mature thrombus. In some instances, the occlusion 106 can be referenced as thrombus, a stenosis, and/or a lesion. Generally, the composition of the occlusion 106 will depend on the type of anatomy being evaluated. Healthier portions of the anatomy 102 may have a uniform or symmetrical profile (e.g., a cylindrical profile with a circular cross-sectional profile). The occlusion 106 may not have a uniform or symmetrical profile. Accordingly, diseased portions of the anatomy 102, with the occlusion 106, will have a non-symmetric and/or otherwise irregular profile. While the anatomy 102 is illustrated in Fig. 1 as having a single occlusion 106, it is understood that the devices, systems, and methods described herein have similar application for anatomy having multiple occlusions.

The intraluminal device 110 includes a pressure sensor 150 coupled to the flexible elongate member 116. As described herein, the pressure sensor 150 can be a capacitive pressure sensor. The pressure sensor 150 can be directly or indirectly coupled to the distal portion 112 of the flexible elongate member 116, proximal of the distal end 113. In some instances, the pressure sensor 150 is positioned less than 10 cm, less than 5, or less than 3 cm from the distal end 113. The pressure sensor 150 is configured to sense external pressure within the lumen 104, such as external pressure associated with the fluid flow within the lumen 104. The pressure sensor 150 generates electrical signals representative of the external pressure and transmits the electrical signals via conductors 140, 142. The conductors 140, 142 individual or collectively can be referenced as electrical conductors, wires, cables, etc. A distal portion of the conductors 140, 142 is mechanically and/or electrically coupled to the pressure sensor 150. As described herein, in some instances, electronic circuitry can be coupled to and/or integrated in the distal portion 112 of the flexible elongate member 116, such as adjacent to, proximal to, and/or integrated in the pressure sensor 150. The electronic circuitry can process electrical signals generated by the sensor and output an electrical signal representative of a sensed pressure. The electrical signals are transmitted from the pressure sensor 150 at the distal portion 112 to the proximal portion 114 of the flexible elongate member 116. The conductors 140, 142 extend along the length of the flexible elongate member 116, from the distal portion 112 to the proximal portion 114. The conductors 140, 142 can carry electrical signals from the PIM 119 and/or the computer 120 to the pressures sensor 150. The electrical signals transmitted to the pressure sensor 150 can be power to activate and operate the pressure sensor 150 and/or control signals to control operation of the pressure sensor 150.

In some embodiments, the intraluminal device 110 includes only two conductors 140, 142. Two conductors 140, 142 can be referred to as a bifilar cable in some instances. Use of only two conductors 140, 142 advantageously minimizes the amount of space taken up by conductors within the intraluminal device 110, compared to larger numbers of conductors. The space within the intraluminal device 110 that is made available by using only two conductors 140, 142 can be advantageously utilized for other components of the intraluminal device 110, such as by making some components larger or adding additional components that provide different functionality while maintaining the same outer diameter of intraluminal device 110. In other instances, the outer diameter of the intraluminal device 110 can be made smaller. In other embodiments, any number of conductors can extend along the length of the flexible elongate member 116 between the connector 117 and the pressure sensor 150, including between one and ten conductors. It is understood that some portion or length of the conductors 140, 142 may be bare while other portions of the conductors 140, 142 may be insulated and/or shielded. For example, a distal end and a proximal end of the conductors 140, 142 can be bare to allow for mechanical and/or electrical interconnection with other components (e.g., by soldering). As shown in the illustrated embodiment of Fig. 2, the conductors 140, 142 can extend linearly along portion(s) of the length of the flexible elongate member and in a spiral or helical configuration along other portions (e.g., around the core wire 136a).

While electrical signals are mentioned, it is understood that the signals representative of the external pressure applied on the pressure sensor 150 can be any suitable signal type, such as optical signals, radio frequency signals, etc. In lieu of or in addition to conductors 140, 142, any suitable communication pathway(s) or communication line(s), wired or wireless, can be implemented in the intraluminal device 110, including an optical fiber, a fiber optical cable, wireless transmission via an antenna integrated in and/or coupled to the distal portion 112 or the proximal portion 114 of the flexible elongate member 116, etc. Generally, the pressure sensor 150 can be any suitable functional device, such as one or more electronic, optical, or electro-optical components. For example, the functional device can be a pressure sensor, a flow sensor (velocity and/or volume), a temperature sensor, an imaging element, an optical fiber, an ultrasound transducer, a reflector, a mirror, a prism, an optical coherence tomography (OCT) element, an ablation element, an RF electrode, a conductor, and/or combinations thereof.

The intraluminal device 110 includes a connector 117 at or adjacent to the proximal portion 114 of the flexible elongate member 116. In some instances, the connector 117 forms part of the proximal end 115 of the flexible elongate member 116. For example, as shown in Fig. 2, the proximal end 115 can be shaped into feature that facilitates alignment and connection of the flexible elongate member to the connector 118. In other instances, the connector 117 is spaced from the proximal end 115 of the flexible elongate member 116. The connector 117 is configured to facilitate communication between the intraluminal device 110 and another device. More specifically, the connector 117 is configured to facilitate communication of data obtained by the pressure sensor 150 to another device, such as the PIM 119 and/or the computer 120. Accordingly, in some embodiments, the connector 117 is an electrical connector that provides an electrical connection to the conductors 140, 142. A proximal portion of the conductors 140 is electrically and/or mechanically coupled to the connector 117. As shown in Fig. 2, for example, the connector 117 includes one or more conductive connector segments 111 encircling the flexible elongate member 116. The connector segments 111 can be rings formed of metal or metal alloy, or can be conductive ink. Each conductor 140, 142 can be directly or indirectly in communication to a respective connector segment 111. For example, the flexible elongate member 116 can include conductive ribbons 141 embedded in a polymer layer at the proximal portion 114. Each conductor 140, 142 can be directly in communication to a respective conductive ribbon 141, which is in turn directly in communication with a respective connector segment 111. In other embodiments, the connector 117 is an optical connector and/or other suitable wired or wireless communication pathway. In some instances, the connector 117 is configured to provide a physical connection to another device, either directly or indirectly. In other instances, the connector 117 is configured to facilitate wireless communication between the intraluminal device 110 and another device. In yet other instances, the connector 117 facilitates both physical and wireless connection to another device. In some instances, the connector 117 is a separate component secured to the flexible elongate member 116 in some instances. In other instances, the connector 117 is integrally formed as a part of the flexible elongate member 116.

The intraluminal device 110 can include a housing 130 in which the pressure sensor 150 is disposed. The housing 130 can be referenced as a sensor housing in some embodiments. The housing 130 includes an opening 132 that exposes the pressure sensor 150 to the fluid within the lumen 104 of the anatomy 102. The housing 130 is coupled to the flexible elongate member 116. For example, the housing 130 can be directly or indirectly coupled to the distal portion 112 of the flexible elongate member 116, proximal of the distal end 113. In some instances, the housing is positioned less than 10 cm, less than 5, or less than 3 cm from the distal tip 105. The housing 130 can be a structure formed of any suitable material, such as a metal, metal alloy, plastic, and/or polymer. In some instances, the housing is a separate component secured to the flexible elongate member 116 in some instances. In other instances, the housing is integrally formed as a part of the flexible elongate member 116. The housing 130 can be tubular structure including a lumen in which one or more components, including the pressure sensor 150 are positioned.

The system 100 can include a connector 118 that is removably coupled to intraluminal device 110. For example, the connector 118 can be mechanically and/or electrically connected to the connector 117 at the proximal portion 114 of the flexible elongate member 116 at the start of a pressure-sensing procedure and disconnected at the end of the procedure. The connector 118 can facilitate communication of data between intraluminal device 110 (e.g., the pressure sensor 150 via the conductors 140, 142 and the connector 117) and another device, such as the PIM 119 and computer 120. For example, the connector 118 can be in direct communication with the PIM 119. Accordingly, in some embodiments, the connector 117 is an electrical connector that provides an electrical connection to the connector 117 of the intraluminal device 110. In other embodiments, the connector 118 is an optical connector and/or other suitable wired or wireless communication pathway. In some instances, the connector 118 is configured to provide a physical connection to another device, either directly or indirectly.

The PIM 119 of the system 100 includes electronic circuitry associated with signals to and from the pressure sensor 150 and the computer 120. In that regard, the PIM 119 is communicatively coupled to the intraluminal device 110 and the computer 120. For example, the PIM 119 can perform processing of the electrical signals received from the pressure sensor 150. The PIM 119 can transmit power and/or control signals to the pressure sensor 150 from the PIM itself and/or the computer 120. The PIM 119 can include one or more processor and memory in some instances to implement hardware and/or software associated with signals to and from the pressure sensor 150 and the computer 120. In some instances, the system 100 does not include the PIM 119, and the intraluminal device 110 communicates with the computer 120 without the PIM 119.

The computer 120 is communicatively coupled to the PIM 119 and/or the intraluminal device 110 (e.g., the pressure sensor 150 and/or electronic circuitry integrated in the pressure sensor 150 and/or the flexible elongate member 116). The computer 120 is generally representative of any one or multiple computing devices suitable for processing and analyzing the data obtained the pressure sensor 150 and/or controlling the operation of the pressure sensor 150. The computer 120 includes one or more processors 122 in communication with any suitable memory 124. The memory 124 can be referenced as a non-transitory computer readable storage medium in some instances. It is understood that any steps related to data acquisition, data processing, instrument control, and/or other processing or control aspects of the present disclosure may be implemented by the computer 120 using corresponding instructions stored on or in the memory 124 and executed by the processor 122. In some instances, the computer 120 is a console device. In some instances, the computer 120 is portable (e.g., handheld, on a rolling cart, etc.).

Generally, the computer 120 is configured to receive the electrical signals from the pressure sensor 150 representative of the sensed pressure within the lumen 104 of the anatomy 102. The computer 120 processes the electrical signals to generate a pressure value. The pressure value can be the sensed pressure within the lumen 104. In some instances, the pressure value can be a pressure ratio calculated by computer 120 based on the sensed pressure, such as a fractional flow reserve (FFR) value, an instantaneous wave-free ratio (iFR) value, a Pd/Pa (distal pressure/aortic pressure) value, and/or other pressure ratio value. The computer 120 can be in communication with another pressure sensor, such as an aortic pressure sensor, a pressure-sensing guidewire, or a pressure-sensing catheter. The computer 120 can calculate the pressure ratio based on the sensed pressure from the pressure sensor 150 and the sensed pressure from the other pressure sensor. The computer 120 generates a visual representation based on the pressure value and outputs the visual representation to the display 125. The visual representation can include a numerical value, symbol, plot, graph, chart, image, and/or other suitable graphical representations of the sensed pressure and/or the calculated pressure ratio. The display 125 can be any suitable monitor, such as a standalone device or can be integrated in a housing of the computer 120.

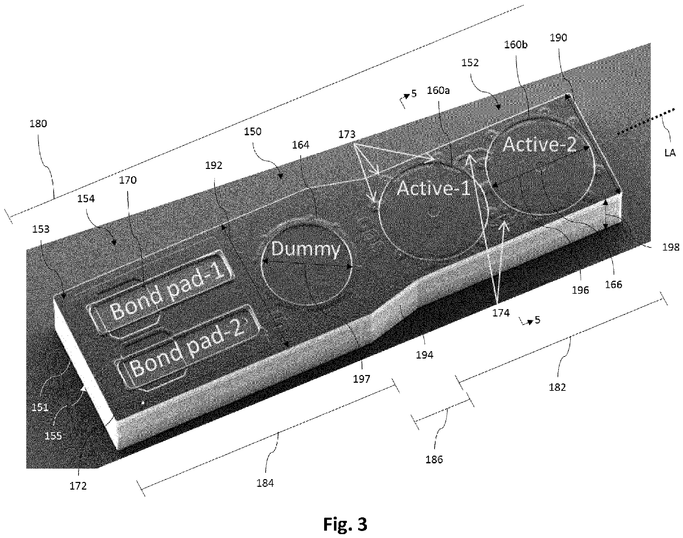

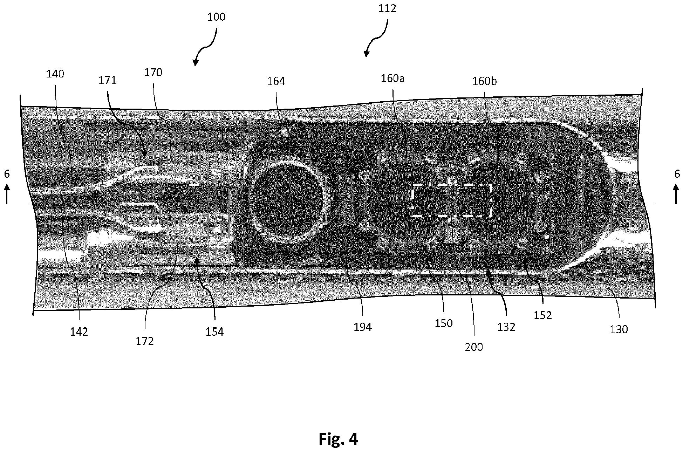

Referring now to Figs. 3 and 4, shown therein is the pressure sensor 150, according to embodiments of the present disclosure. Fig. 3 is a diagrammatic perspective view of the pressure sensor 150. For example, Fig. 3 is a scanning electronic microscope (SEM) image of the pressure sensor 150 (e.g., after the pressure sensor 150 has been singulated during fabrication). Fig. 4 is a diagrammatic top view of the pressure sensor 150 disposed within the housing 130. The pressure sensor 150 in the illustrated embodiment is a capacitive pressure sensor. The pressure sensor 150 includes three capacitive cells formed in a substrate 151: two active cells 160a, 160b and a dummy cell 164. The active cells 160a, 160b are electrically active to generate electrical signals representative of the external pressure applied to the pressure sensor 150 by the fluid within the lumen 104 of the anatomy 102. The dummy cell 164 is electrically inactive such that no electrical signal representative of the external pressure is generated. In an exemplary embodiment, each pressure sensor 150 includes only three capacitive cells: the two active cells 160a, 160b and the dummy cell 164. In some embodiments, the pressure sensor 150 can be referenced as a microelectromechanical system (MEMS) pressure sensor. In that regard, the components of the pressure sensor 150, such as the active cells 160a, 160b, the dummy cell 164, and an integrated circuit 200, can be formed according to semiconductor processes (e.g., similar to processes for fabricating capacitive micromachined ultrasound transducers or CMUTs). Structurally, the active cells 160a, 160b and the dummy cell 164 are similar or identical capacitive cells formed according to the same processes. The active cells 160a, 160b are different from the dummy cell 164 in that they are electrically connected to and/or otherwise in communication with other components of pressure sensor 150 (e.g., the integrated circuit 200 disposed in the substrate 151 below the active cell 160a), while pressure sensor 150 does not include any electrical connection with the dummy cell 164. In other embodiments, the dummy cell 164 is electrically active, electrically connected, and generates electrical signals. In such embodiments, the dummy cell 164 can be used for pressure sensing, flow sensing (velocity and/or volume), temperature sensing, ultrasound transduction (e.g., emitting ultrasound energy and/or receiving ultrasound echoes reflected from anatomy, such as for ultrasound imaging), and/or other suitable uses.

The active cells 160a, 160b include a dimension 196, which may be diameter. In some embodiments, the dimension 196 can be between approximately 100 μm and approximately 170 μm, between approximately 120 μm and approximately 150 μm, and/or between approximately 130 μm and 140 μm, including values such as 130 μm, 133 μm, 135 μm, 137 μm, 140 μm, and/or other suitable values both larger and smaller. The dimension 196 of the active cells 160a, 160b can be equal to one another. Generally, the structure of the active cells 160a, 160b is the identical such that the behavior of the active cells 160a, 160b under the influence of external pressure is identical. In other embodiments, the size, shape, and/or other structural aspects of the active cells 160a, 160b are different from one another. The dimension 196 of the active cells 160a, 160b may be different than a corresponding dimension 197, such as a diameter, of the dummy cell 164. For example, the dimension 197 of the dummy cell 164 may be less than then dimension 196 of the active cells 160a, 160b. In some embodiments, the dimension 197 can be between approximately 100 μm and approximately 170 μm, between approximately 120 μm and approximately 150 μm, and/or between approximately 130 μm and 140 μm, including values such as 127 μm, 130 μm, 133 μm, 135 μm, 137 μm, and/or other suitable values both larger and smaller. In other embodiments, the dimension 196 of the active cells 160a, 160b is equal to the dimension 197 of the dummy cell 164.

The substrate 151 includes a surface 153 on which the active cells 160a, 160b, the dummy cell 164, and the bonds pads 170, 172 are formed, and an opposite surface 155. As shown in Fig. 4, the pressure sensor 150 is arranged within the housing 130 such that the active cells 160a, 160b and the dummy cell 164 are oriented towards the opening 132 in the housing 130. For example, the surface 153 of the substrate 151 faces the opening 132. In this manner, the pressure sensor 150 (e.g., the active cells 160a, 160b) is exposed to the external pressure of the fluid flow within the lumen 104. Adhesive and/or potting material 171 can be positioned within the housing 130 over the proximal portion 154 of the pressure sensor 150, to cover the soldered connection between the distal ends of the conductors 140, 142 and the bond pads 170, 172.

In the illustrated embodiment, the active cells 160a, 160b and the dummy cell 164 are arranged longitudinally along the pressure sensor 150 and/or the substrate 151. For example, the active cells 160a, 160b and the dummy cell 164 are disposed in the substrate 151 adjacent to or proximate to one another along a dimension 180, such as a length, of the pressure sensor 150 and/or along the longitudinal axis LA. The active cells 160a, 160b and the dummy cell 164 can be arranged in a line. For example, when the longitudinal axis LA is a central longitudinal axis of the pressures sensor 150, the mid-points of the active cells 160a, 160b and the dummy cell 164 are disposed along the central longitudinal axis. In other embodiments, the active cells 160a, 160b and the dummy cell 164 are arranged in different configurations. For example, one or more of the active cells 160a, 160b and the dummy cell 164 are positioned side-by-side laterally and/or laterally offset from one another or from the central longitudinal axis.

The pressure sensor 150 and/or the substrate 151 includes a distal portion 152 and a proximal portion 154. The active cells 160a, 160b can be formed at the distal portion 152. Bond pads 170, 172 can be formed in the substrate 151 at the proximal portion 154. The bond pads 170, 172 extend longitudinally along the along the pressure sensor 150 and/or the substrate 151. The bond pads 170, 172 are arranged side-by-side laterally in the illustrated embodiment, in a direction perpendicular to the longitudinal axis LA (e.g., a transverse dimension of the pressure sensor 150 and/or the substrate 151). Different configurations for the bond pads 170, 172 in different embodiments are contemplated. The bond pads 170, 172 includes a conductive material that is directly or indirectly in communication with the active cells 160a, 160b as a result of conductive traces or other conductive signal pathways formed in the substrate 151. For example, the bond pads 170, 172 can be in communication with the integrated circuit 200. The distal end of the conductors 140, 142 are mechanically and/or electrically coupled to the bond pads 170, 172, respectively, thereby establishing electrical communication between the conductors 140, 142 and the active cells 160a, 160b. For example, the distal end of the conductors 140, 142 are soldered to the bond pads 170, 172, respectively, thereby mechanically coupling and establishing communication between the conductors 140, 142 and the bond pads 172. The dimensions of the bond pads 170, 172 (e.g., a length and a width) provide space for the conductors 140, 142, and the solder. The dummy cell 164 can be located at least partially in the proximal portion 154 and/or in a middle portion between the distal portion 152 and the proximal portion 154.

The substrate 151 is formed into any suitable size and shape such that the pressure sensor 150 can be implemented in the intraluminal device 110. In that regard, the substrate 151 is sized and shaped, structurally arranged, and/or otherwise configured to be disposed within the housing 130, coupled to the intraluminal device 110 and/or positioned within the lumen 104 of the anatomy 102. In the illustrate embodiment, the pressure sensor 150 and/or the substrate 151 is formed into a bottle shape, with the distal portion 152 being the neck of the bottle, the proximal portion 154 being the body of the bottle, and a transition 194 between the distal portion 152 and the proximal portion 154 being the shoulder of the bottle. In other embodiments, the pressure sensor 150 is formed in any suitable geometric or non-geometric shape. The substrate 151 can be any suitable semiconductor material, such as a silicon (Si) substrate or a germanium (Ge) substrate. In some embodiments, the substrate 151 may include a compound semiconductor such as silicon carbide (SiC), silicon germanium (SiGe), silicon germanium carbide (SiGeC). In some implementations, the substrate 151 may be a silicon on insulator (SOI) substrate.

As shown in Fig. 3, the substrate 151 of the pressure sensor 150 includes the dimension 180, which may be a length of the substrate 151. In some embodiments, the dimension 180 can be between approximately 500 μm and approximately 1000 μm, between approximately 700 μm and approximately 800 μm, and/or between approximately 725 μm and 775 μm, including values such as 700 μm, 725 μm, 740 μm, 750 μm, 760 μm, 775 μm, 800 μm, and/or other suitable values both larger and smaller. The substrate 151 includes a dimension 198, which may be thickness or depth of the substrate 151. In some embodiments, the dimension 198 can be between approximately 50 μm and approximately 100 μm, between approximately 60 μm and approximately 80 μm, and/or between approximately 65 μm and 75 μm, including values such as 60 μm, 65 μm, 68 μm, 70 μm, 72 μm, 75 μm, 80 μm, and/or other suitable values both larger and smaller. The dimension 198 can be constant along the entire length of the substrate 151, or can vary smoothly or sharply (e.g., different dimension 198 in the distal portion 152, the proximal portion 154, and/or the transition 194).

The distal portion 152 of the substrate 151 includes a dimension 190, which may be width of the distal portion 152. In some embodiments, the dimension 190 can be between approximately 120 μm and approximately 200 μm, between approximately 140 μm and approximately 180 μm, and/or between approximately 155 μm and 165 μm, including values such as 150 μm, 155 μm, 158 μm, 160 μm, 162 μm, 165 μm, 170 μm, and/or other suitable values both larger and smaller. The proximal portion 154 of the substrate 151 includes a dimension 192, which may be width of the proximal portion 154. In some embodiments, the dimension 192 can be between approximately 150 μm and approximately 250 μm, between approximately 175 μm and approximately 225 μm, and/or between approximately 190 μm and 210 μm, including values such as 190 μm, 195 μm, 198 μm, 200 μm, 202 μm, 205 μm, 210 μm, and/or other suitable values both larger and smaller. In some embodiments, the dimension 190 of the distal portion 152 is smaller than the corresponding dimension 192 of the proximal portion 154. For example, the proximal portion 154 is wider than the distal portion 152. The transition 194 transitions from the larger dimension 192 of the proximal portion 154 to the smaller dimension 190 of the distal portion 152. In the illustrate embodiment, the transition 194 is linear. In other embodiments, the transition 194 may be curved.

The distal portion 152 of the substrate 151 includes a dimension 182, which may be length of the distal portion 152. In some embodiments, the dimension 182 can be between approximately 250 μm and approximately 350 μm and/or between approximately 375 μm and approximately 425 μm, including values such as 290 μm, 300 μm, 302 μm, 310 μm, and/or other suitable values both larger and smaller. The proximal portion 154 of the substrate 151 includes a dimension 184, which may be length of the proximal portion 154. In some embodiments, the dimension 184 can be between approximately 340 μm and approximately 400 μm and/or between approximately 350 μm and approximately 390 μm, including values such as 360 μm, 370 μm, 373 μm, 380 μm, and/or other suitable values both larger and smaller. The transition 194 of the substrate 151 includes a dimension 186, which may be length of the transition 194. In some embodiments, the dimension 186 can be between approximately 25 μm and approximately 100 μm and/or be-

tween approximately 60 μm and approximately 90 μm, including values such as 70 μm, 80 μm, 90 μm, and/or other suitable values both larger and smaller.

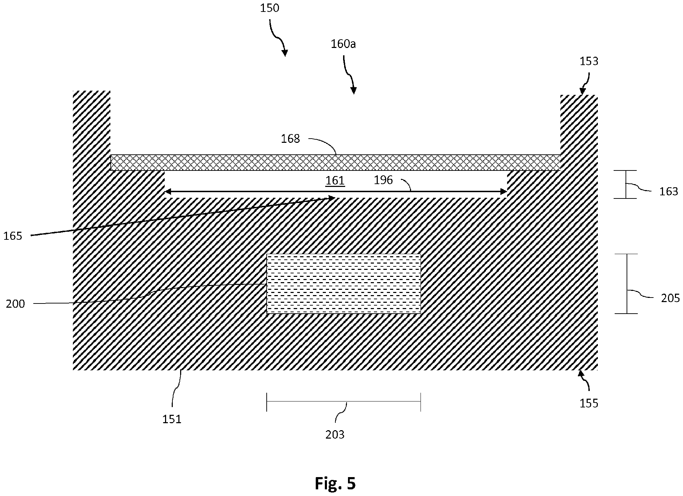

Referring now to Fig. 5, shown therein is a diagrammatic cross-sectional view of the pressure sensor 150 along section line 5-5 in Fig. 3, according to an embodiment of the present disclosure. The pressure sensor 150 includes the integrated circuit 200 electrically coupled to the active cells 160a, 160b. The active cell 160a includes a membrane 168 disposed over a cavity 161 formed in the substrate 151. The cavity can have a dimension 163, such as height, between approximately 300 nm and approximately 400 nm, including values such as 325 nm, 350 nm, 375 nm, and/or other suitable values both larger and smaller. The tolerance of the dimension 163 can be ± 25 nm in an exemplary embodiment. While only the active cell 160a is illustrated in Fig. 5, it is understood that the active cell 160b can have the identical structure and function.

The basic principal of the active cells 160a, 160b is a parallel-plate capacitor between electrodes provided in the membrane 168 and the substrate 151, at a base 165 of the cavity 161. The electrode at the base 165 is fixed, while membrane 168 and/or the electrode in the membrane 168 moves in response to the external pressure applied to the pressure sensor 150. The active cells 160a, 160b transmit electrical signals representative of the change in capacitance resulting from movement of the membrane 168. The integrated circuit 200 receives the electrical signals output by the active cells 160a, 160b. The integrated circuit 200, the PIM 119, and/or the computer 120 use the electrical signals to measure the pressure of the fluid flow within the lumen 104 of the anatomy 102. In an exemplary embodiment, the active cells 160a, 160b are electrically connected to one another in parallel such that the respective capacitances measured by the active cells 160a, 160b is added (e.g. $C_1 + C_2$). In other embodiments, the active cells 160a, 160b can be connected in series or not connected to one another. The dummy cell 164 is not electrically coupled to the integrated circuit 200 and does not transmit the electrical signals representative of the sensed pressure. The dimension 196 of the active cells 160a, 160b can be a dimension (e.g., diameter) of the membrane 168 in some embodiments, n some embodiments, the active cells 160a, 160b and the dummy cell 164 are annular capacitive cells. For example, as shown in Fig. 3, the active cells 160a, 160b and the dummy cell 164 include a pillar 166 located within the cavity 161. For example, the pillar 166 can be located in the center of the cavity 161. The pillar 166 extends vertically from the substrate 151 forming the base 165 of the cavity 161 (Fig. 5). For example, the cavity 161 has an annular shape as a result of the pillar 166. The pillar 166 can contact the membrane 168 and prevent the membrane 168 from contact the substrate 151 forming the based on the cavity 161. In other embodiments, the active cells 160a, 160b and the dummy cell 164 are circular or cylindrical capacitive cells. For example, the cavity 161 has a cylindrical shape, with a circular cross-sectional profile. The active cells 160a, 160b can be operated in collapse/contact mode or conventional, non-collapse mode. The pressure sensor 150 can includes features similar to those described in European Application No. EP18188185.5, filed August 9, 2018, and titled "Pressure Sensing with Capacitive Pressure Sensor".

As shown in Fig. 3, the pressure sensor 150 includes vias 174 providing a communication pathway for electrical signals between the active cells 160a, 160b and the integrated circuit 200. The integrated circuit 200 can be referenced as electronic circuitry in some instances. The integrated circuit 200 can include any suitable electronics configured to process the electrical signals output by the active cells 160a, 160b. For example, the integrated circuit 200 can be an application specific integrated circuit (ASIC). The integrated circuit 200 can also be configured to provide electrical signals (e.g., power and/or control signals) to the active cells 160a, 160b.

The integrated circuit 200 is directly or indirectly in communication with the conductors 140, 142. For example, the integrated circuit 200 is electrically coupled to the conductors 140, 142 via the bonds pads 170, 172, conductive traces, and/or other conductive signal pathways formed in the substrate 151. In that regard, the bond pads 170, 172 are electrically coupled to the integrated circuit 200. The integrated circuit 200 transmits an electrical signal via at least one of the conductors 140, 142. The integrated circuit 200 is configured to output an electrical signal representative of a sensed pressure at the active cells 160, 160b. In an exemplary embodiment, the integrated circuit 200 outputs an alternating current (AC) signal. In that regard, one of the conductors 140, 142 can be at electrical ground (e.g., 0 V) and the other conductor can carry an electrical signal providing power to the active cells 160a, 160b and/or the integrated circuit 200 (any suitable voltage, such as a 2.5 V, 3.0 V, and/or other values both larger and smaller). The power supply voltage can be transmitted to the pressure sensor 150 by the PIM 119 and/or the computer 120. In an exemplary embodiment, the integrated circuit 200 can generally behave as an *RC* oscillator with an oscillation frequency *f* proportional to $\frac{1}{RC}$. The oscillation frequency can also be referenced as an output frequency in some instances. The oscillation frequency is based on the capacitances of the two of the active cells 160a, 160b. When the active cells 160a, 160b are electrically connected in parallel, the electrical signal output by the integrated circuit 200 has an oscillation frequency $f \sim \frac{1}{R(C_1+C_2)}$. When the external pressure is exerted on the active cells 160a, 160b, causing the membrane 161 to deflect towards the base 165 of the cavity 161, the capacitances increase and the oscillation frequency decreases. With less pressure or no pressure is exerted on the active cells 160a, 160b, the membrane 161 deflects away from the base 165, causing the capacitances to decrease and the oscillation frequency to increase. The oscillations in the electrical signal output by the integrated circuit 200 are provided on top of the power supply voltage (e.g., 2.5 V, 3 V) carried by one of the conductors 140, 142. The conductor 140 and/or 142 can transmit the electrical signal representative of the sensed pressure from the capacitive pressure sensor 150 at the distal portion 112 of the flexible elongate member 116 to the connector 117 at the proximal portion 114 of the flexible elongate member 116. The PIM 119 and/or the computer 120, which are in communication with the connector 117, detect the frequency in the electrical signal carried by one of the conductors 140, 142, resulting from the output of the integrated circuit 200. For example, the oscillation frequency at the bond pads 170, 172 is representative of the sensed pressure at the active cells 160a, 160b. The PIM 119 and/or computer 120 determines the sensed pressure at the active cells 160a, 160b based on the electrical signal carried by the conductors 140, 142. For example, the computer 120 utilizes a calibration curve describing the relationship between oscillation frequency and the sensed pressure. In some instances, the calibration curve is a generally smooth curve with a negative slope. That is, the lower the oscillation frequency, the higher the sensed pressure. Likewise, the higher the oscillation frequency, the lower the sensed pressure. The computer 120 can utilize any suitable mathematical relationship and/or function between oscillation frequency and the sensed pressure to determine the sensed pressure based on the electrical signal output by the integrated circuit 200.

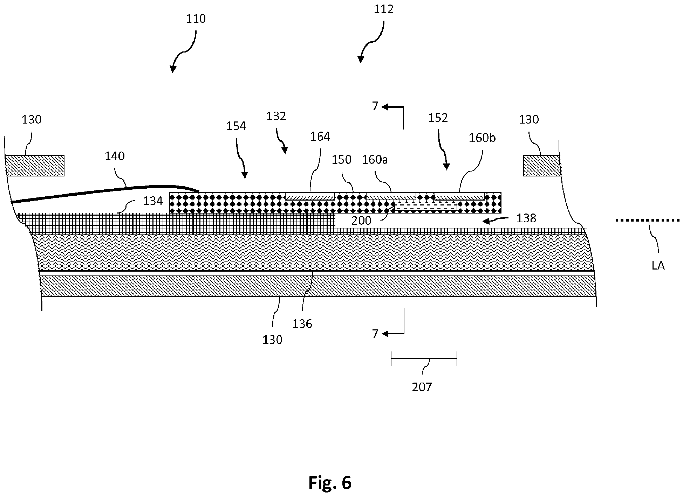

The integrated circuit 200 is disposed in the substrate 151. For example, the integrated circuit 200 can be disposed in the distal portion 152 of the substrate 151. In the illustrated embodiment, the integrated circuit 200 is completely surrounded by the substrate 151. In other embodiments, the integrated circuit 200 can be disposed in the substrate 151 at the surface 153 or the surface 155. For example, a surface of the integrated circuit 200 can extend continuously with the surface 153 or the surface 155. The pressure sensor 150 may only include one integrated circuit 200, which may be disposed in the substrate 151 below the active cell 160a and/or the active cell 160b. In an exemplary embodiment, the integrated circuit 200 is sized and shaped such that it is partially positioned under each of the active cells 160a, 160b and a region between active cells 160a, 160b. The integrated circuit 200 can be symmetrically disposed below the active cells 160a, 160b. In that regard, the integrated circuit 200 has a midpoint that is co-located with a midpoint between the active cells 160a, 160b. For example, as shown in Figs. 4 and 6, proximal and distal portions of the integrated circuit 200 can longitudinally overlap with the active cells 160, 160b equally. For example, with respect to the orientation shown in Fig. 4, the top and bottom edges of the integrated circuit 200 can be spaced equally from the top and bottom edges of the distal portion 152 of the substrate 151. The integrated circuit 200 can be any suitable geometric or non-geometric shape. For example, the integrated circuit 200 can be a rectangular prism. In some embodiments, the pressure sensor 150 includes two or more integrated circuits 200. In some embodiments, the pressure sensor 150 is laterally or longitudinally adjacent to at least one of the active cells 160a, 160b.

The integrated circuit 200 includes a dimension 203, such as a width of the integrated circuit 200. In some embodiments, the dimension 203 can be between approximately 50 $\mu$m and approximately 60 $\mu$m, including values such as 53 $\mu$m, 55 $\mu$m, 57 $\mu$m, and/or other suitable values both larger and smaller. In such embodiments, the dimension 203 of the integrated circuit 203 can be smaller than the dimension 196 of the active cell 160a. In that regard, the dimension 196 of the active cell 160 can be a width or a diameter of the cavity 161. The integrated circuit 200 includes a dimension 205, such as a height of the integrated circuit 200. In some embodiments, the dimension 205 can be between approximately 5 $\mu$m and approximately 25 $\mu$m and/or between approximately 10 $\mu$m and approximately 20 $\mu$m, including values such as 10 $\mu$m, 15 $\mu$m, 20 $\mu$m, and/or other suitable values both larger and smaller. In an exemplary embodiment, the dimension 205 of the integrated circuit 203 is larger than the dimension 163 of the cavity 161. A dimension 207 (Fig. 6) of the integrated circuit 200, such as length (e.g., along the longitudinal axis LA) can be between approximately 130 $\mu$m and approximately 160 $\mu$m and/or between approximately 140 $\mu$m and approximately 145 $\mu$m, including values such as 141 $\mu$m, 143 $\mu$m, 144 $\mu$m, 145 $\mu$m, and/or other suitable values both larger and smaller.

Referring again to Fig. 3, the substrate 151 includes etching holes 173 surrounding the active cells 160a, 160b, which result from the semiconductor processes used to form the active cells 160a, 160b. The etching holes 173 are used in the sacrificial etching process that creates the cavity 161 illustrated in Fig. 5. After sacrificial etching, the membrane 168 is free hanging and only supported at its rim. The next step is the deposition of a plug layer that closes the etching holes 173 and seals the cavity 161. The cavity 161 must be emptied to create active cells 160a, 160b that can measure pressure. A cavity need not be emptied for the dummy cell 164 because the dummy cell 164 is not used to measure pressure in an exemplary embodiment. There can be several reasons for omitting the etching holes 173 from the dummy cell 164. There is a small risk that the membrane 168 comes loose. The opening of a cavity of the dummy cell 164 can create some issues, for example, in vivo release of particles in the blood vessel if a free-hanging membrane is broken/damaged during a clinical procedure. Because the active cells 160a, 160b are in electrical communication with other components, any rupture of the membrane 168 of the active cells 160a, 160b can be electrically detected via the electrical signal output by the pressure sensor 150. Unlike the active cells 160a, 160b, rupture of the membrane 168 of the dummy cell 164 cannot be electrically detected because the dummy cells 164 is not in electrical communication with other components. It can be safer that the membrane 168 of the dummy cell 164 not be free-hanging. By omitting the etching holes 173, undetectable or hardly detectable in vivo release of particles can be advantageously prevented. In an exemplary aspect, the inventors have found the yield to advantageously increase when a cavity is not emptied for the dummy cell 164. Because the main proximity effects on deposition, lithography, and etching are independent of the present of the etching holes 173, the etching holes 173 can be omitted for the dummy cell 164. In some embodiments, a cavity of the dummy cell 164 is emptied, and the dummy cell 164 includes etching holes 173.

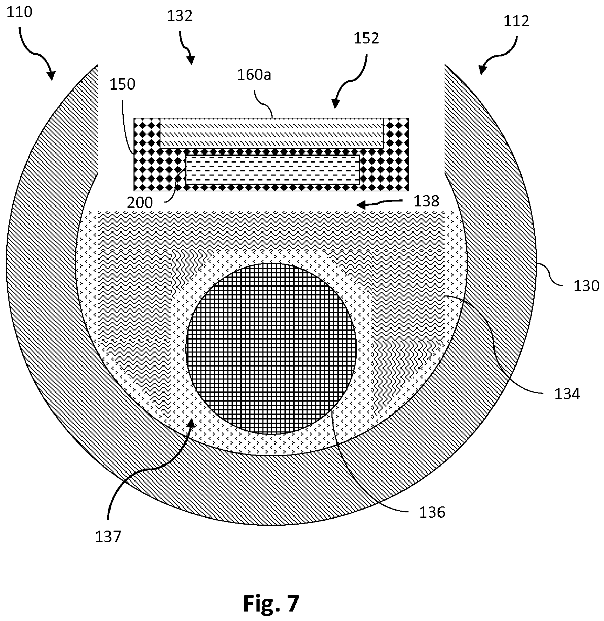

Referring now to Figs. 6 and 7, shown therein is the distal portion 112 of the intraluminal device 110. Fig. 6 is diagrammatic cross-sectional view along section line 6-6 in Fig. 4. Fig. 7 is a diagrammatic cross-sectional view along section line 7-7 in Fig. 6. Figs. 6 and 7 illustrate the pressure sensor 150 disposed within the housing 130. The housing 130 includes a mount 134 disposed within the housing 130. The mount 134 can be referenced as a sensor mount in some instances. The mount 134 is any suitable structure configured to support and/or be coupling point for other components of the intraluminal device 110 (e.g., the pressure sensor 150). The mount 134 can be a structure formed of any suitable material, such as a metal, metal alloy, plastic, and/or polymer. The mount 134 can be mechanically coupled to the housing 130 using, e.g., an adhesive 137. The core wire 136 can extend longitudinally through the housing 130 and/or mount 134. For example, the housing 130 and/or mount 134 can at least partially surround the core wire 136. The core wire 135 can be mechanically coupled to the mount 134 and/or the housing 130 using, e.g., the adhesive 137.

The proximal portion 154 of the pressure sensor 150 can be coupled to the mount 134. Generally, the proximal portion 154 can be directly or indirectly coupled to the mount 134 and/or the housing 130 in which the mount 134 is positioned. In general, mechanical coupling, attachment, connection, and/or securing between components can include direct or indirect fastening where one or more other components are disposed between coupled components. For example, components can be mechanically coupled using an adhesive, mechanical fasteners, welding, and/or other suitable attachment. The dummy cell 164 can be disposed mostly in the proximal portion 154. Fig. 6 shows the conductor 140 electrically and/or mechanically coupled to the pressure sensor 150 at the proximal portion 154, at bond pad 170, for example.

The distal portion 152 of the pressure sensor 150 is cantilevered, with a gap 138 between the pressure sensor 150 and the mount 134. Because it is cantilevered, the distal portion 152 is advantageously isolated from any forces experienced by the intraluminal device 110, the housing 130, and/or the mount 134 resulting from the intraluminal device 110 navigating the anatomy 102 (e.g., deformation of the intraluminal device 110, the housing 130, and/or the mount 134 caused by bending in tortuous vasculature or contacting tissue while crossing the occlusion 106). The distal portion 152 is also advantageously isolated from any forces experienced by the pressure sensor 150 during assembly of the intraluminal device 110, such as when the proximal portion 154 is coupled to the mount 134. In the illustrated embodiment, the active cells 160a, 160b and the integrated circuit 200, are disposed within the cantilevered distal portion 152. Accordingly, the active cells 160a, 160b and the integrated circuit 200 are advantageously free from the influence of any forces that may adversely impact operation (e.g., the output signals) of the active cells 160a, 160b and the integrated circuit 200 to measure the pressure of the fluid within the lumen 104. Instead, the active cells 160a, 160b experience only the external pressure of the fluid within the lumen 104. In that regard, the active cells 160a, 160b are exposed to the fluid within the lumen 104 through the opening 132 in the housing 130. Any forces associated with deformation of the intraluminal device 110, the housing 130, and/or the mount 134 are experienced by the dummy cell 164, which is not electrically active and is not involved in measuring pressure.

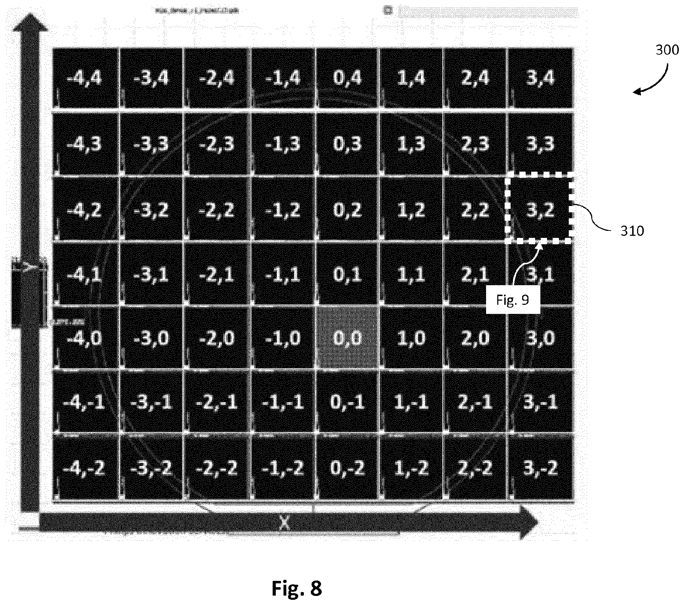

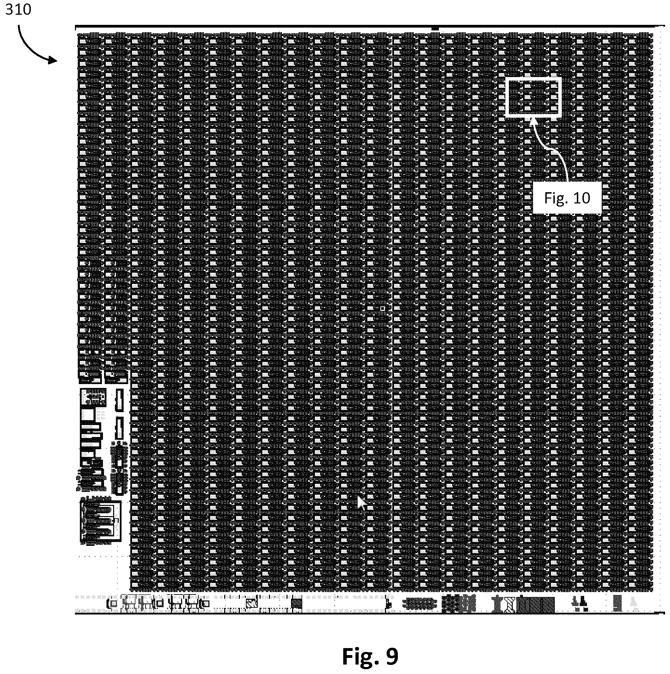

Referring now to Figs. 8 and 9, shown therein are structures during fabrication of the pressure sensor 150. Fig. 8 is a diagrammatic top view of a wafer 300, according to embodiments of the present disclosure. Fig. 9 is a diagrammatic top view of a die 310, according to embodiments of the present disclosure. An exemplary die 310 is indicated in the wafer 300 of Fig. 8. The wafer 300 is a thin slice of semiconductor material. In that regard, the substrate 151 of the pressure sensor 150 is a portion of the wafer 300. The wafer 300 can have be any suitable diameter, such 6", 8", 12", and/or other suitable values both larger and smaller. The wafer 300 includes any suitable number of dies 310. For example, in the illustrated embodiment, the wafer 300 includes fifty-six dies 310. In an exemplary embodiment, approximately one thousand pressure sensors 150 are fabricated on each die 310.

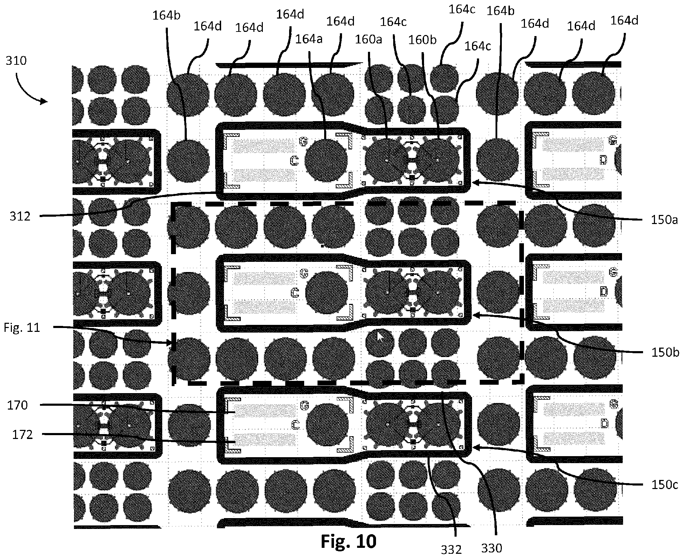

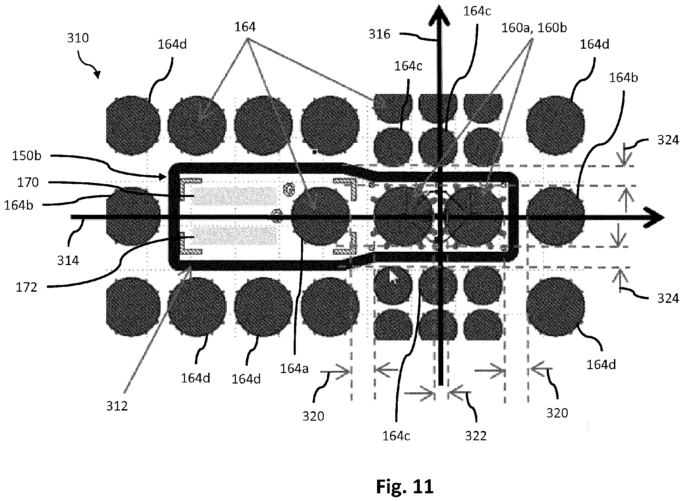

Referring now to Figs. 10 and 11, shown therein are diagrammatic top views of a portion of the die 310 including one or more pressures sensors 150 (e.g., the pressure sensors 150a, 150b, and/or 150c). Fig. 10 illustrates an exemplary region of the die 310 indicated in Fig. 9. Fig. 11 illustrates the exemplary region of the die 310 indicated in Fig. 10. Figs. 10 and 11 illustrates the pressure sensors 150 during a stage of fabrication, prior to singulation of the pressure sensors 150 along boundaries 312. In an exemplary embodiment, the fabrication process includes deep reactive ion etching (DRIE) along the boundary 312 to define the size and shape of the substrate 151 and singulate the pressure sensor 150. Other suitable etching, dicing, grinding, thinning, polishing, and/or cutting techniques can be utilized in various embodiments. As described herein, each pressure sensor 150 includes active cells 160a, 160b and bond pads 170, 172.

As shown in Figs. 10 and 11, each die 310 advantageously includes a dense arrangement of capacitive cells, including the active cells 160a, 160b and dummy cells 164a, 164b, 164c, 164d. The active cells 160a, 160b and the dummy cells 164a are formed in the die 310 within the boundaries 312 of the pressure sensors 150. Dummy cells 164b, 164c, 164d are formed in the die 310 outside of the boundaries 312. The diameters of the dummy cells 164a, 164b, 164c, 164d can be the same or different from one another. The diameters of the dummy cells 164a, 164b, 164d can be equal to one another. In the illustrated embodiment, the diameter of dummy cells 164c is smaller than the diameters of the dummy cells 164a, 164b, 164d. The smaller diameter of the dummy cells 164c advantageously allows for them to be positioned closer to one another and to the active cells 160a, 160b. The dummy cells 164a, 164b, 164c, 164d typically have the similar dimensions as the active cells 160a, 160b, but the dimensions of the dummy cells 164a, 164b, 164c, 164d can be different to provide a more optimal layout. The dummy cells 164a, 164b, 164c, 164d typically resemble the active cells 160a, 160b as good as possible. The presence of the boundary 312 for DRIE prevents one or more of the dummy cells 164a, 164b, 164c, 164d from having identical dimensions to the active cells 160a, 160b. In an exemplary embodiment, the diameter can vary from 90 $\mu$m for the dummy cells 164c to 133 $\mu$m for the dummy cells 164a, 164b, and/or 164d.

In that regard, the dummy cells 164a, 164b, 164c, 164d surround the active cells 160a, 160b. The dummy cells 164c are positioned above and below every pair of active cells 160a, 160b in the die 310. The dummy cells 164a are positioned to the left of the active cells 160a, 160b. The dummy cells 164b are positioned to the right of the active cells 160a, 160b. The dummy cells 164d are positioned diagonally of the active cells 160a, 160b. The directional descriptions such as above, below, left, right, and diagonal are used with reference to the orientation and arrangement of components in Fig. 10. It is understood that different directional descriptions can be used with different orientations and arrangements. The dummy cells 164a, 164b, 164c, 164d can be arranged in grid-like pattern around the active cells 160a, 160b. The dummy cells 164d are disposed between vertically spaced pressure sensors in the die 310 (e.g., between the pressure sensor 150a and the pressure sensor 150b in Fig. 10). The dummy cells 164b are disposed between laterally spaced pressure sensors in the die 310 (e.g., between the pressure sensor 150a and the pressure sensors to the left and right of the pressure sensor 150a in Fig. 10).

The dense arrangement of capacitive cells in the die 310 advantageously ensures uniformity in the active cells 160a, 160b. For example, compared to one another, the active cell 160a and the active cell 160b of the pressure sensor 150a respond similarly or identically to the external pressure of the fluid within the lumen 104 of the anatomy 102. Likewise, the active cells 160a, 160b of the pressure sensor 150a and those of the pressure sensors 150b, 150c, respond similarly or identically to the external pressure of the fluid within the lumen 104. Additionally, the active cells 160a, 160b of the pressure sensor 150 and those of a pressure sensor fabricated on a different die 310 and/or a different wafer 300 respond similarly or identically to the external pressure of the fluid within the lumen 104. Moreover, the active cells 160a, 160b of the pressure sensor 150 and those of a pressure sensor fabricated in a different batch (e.g., at a different time, on a different wafer 300) respond similarly or identically to the external pressure of the fluid within the lumen 104. As a result, the sensed pressure is consistent across all of the active cells 160a, 160b in the same batch and across different batches.

One of the challenges in producing a capacitive pressure sensor is reproducibility of the manufacturing method with the same sensor sensitivity from one MEMS batch to another. It is well known that the membranes of capacitive cells at the edge of CMUT arrays suffer from non-uniformities such that edge and corner membranes have different mechanical properties due to processing non-uniformity. The present disclosure advantageously addresses this challenge by providing a spatially compact distribution of capacitive cells within the wafer 300 and/or the die 310. This dense arrangement is made possible by including the dummy cells 164a, 164b, 164c, 164d in between and surrounding the active cells 160a, 160b. For example, the distribution of the dummy cells 164a, 164b, 164c, 164d evenly surrounds the active cells 160a, 160b. This arrangement eliminates the possibility that the active cells 160a, 160b are located at the edge or corners of the die 310 and/or the wafer 300, where the processing non-uniformities arise. If any processing non-uniformities arise, they do so only in the dummy cells 164a, 164b, 164c, 164d, which are not electrically active and are not involved in measuring pressure. The dummy cells 164a, 164b, 164c, 164d provide a uniform surrounding for the active cells 160a, 160b, preventing nonuniform behavior of the active cells 160a, 160b during deposition, lithograpy, and etching steps in the fabrication process, known as proximity effects. The dummy cells 164a, 164b, 164c, 164d are provided to advantageously improve processing uniformity. In that regard, the active cells 160a, 160b are always spaced from the corner or edge of the die 310 and/or the wafer 300 by at least one dummy cell 164a, 164b, 164c, 164d. As shown in Fig. 3, for example, the presence of the dummy cell 164a on the singulated pressure sensors 150 is an indication of this advantageously dense arrangement of capacitive cells in the wafer 300 and/or the die 310.

The active cells 160a, 160b are also disposed in a symmetrical environment within the dense arrangement of capacitive cells in the die 310. In that regard, an axis 314 and an axis 316 are shown in Fig. 11, which may be referenced as symmetry axes. In that regard, the active cells 160a, 160b can be symmetrical about the axis 314 and/or the axis 316. The axis 314 is a lateral axis or x-axis. The axis 316 is a vertical axis or y-axis. The active cells 160a, 160b are also symmetrically arranged with respect to the dummy cell 164a to the left and the dummy cell 164b to the right (e.g., along the axis 314). In the regard, the dummy cell 164a is spaced from its nearest active cell 160a by the same pitch 220 as the dummy cell 164b is spaced from its nearest active cell 160b. The pitch 320 can be any suitable value in various embodiments. In some embodiments, the pitch 320 can be between approximately 30 $\mu$m and approximately 70 $\mu$m, between approximately 40 $\mu$m and approximately 60 $\mu$m, and/or between approximately 50 $\mu$m and approximately 55 $\mu$m, including values such as 48 $\mu$m, 50 $\mu$m, 51 $\mu$m, 52 $\mu$m, 53 $\mu$m, 54 $\mu$m, 55 $\mu$m, 57 $\mu$m and/or other suitable values both larger and smaller.

The active cells 160a, 160b are also symmetrically arranged with respect to the dummy cells 164c above and below (e.g., along the axis 316). In the regard, the dummy cells 164c nearest to and above the active cells 160a, 160b (adjacent and/or proximate to a top edge 330 of the pressure sensor 150 in Fig. 10), as well as the dummy cells 160c nearest to and below the active cells 160a, 160b (adjacent and/or proximate to bottom edge 332 of the pressure sensor 150 in Fig. 10), are spaced by the same pitch 224. The pitch 324 can be any suitable value in various embodiments. In some embodiments, the pitch 324 can be between approximately 20 $\mu$m and approximately 60 $\mu$m, between approximately 30 $\mu$m and approximately 50 $\mu$m, and/or between approximately 42 $\mu$m and approximately 46 $\mu$m, including values such as 40 $\mu$m, 42 $\mu$m, 43 $\mu$m, 44 $\mu$m, 45 $\mu$m, 46 $\mu$m, 48 $\mu$m, and/or other suitable values both larger and smaller.

The symmetrical environment in which the active cells 160a, 160b are fabricated additionally prevents any processing non-uniformities. Both the active cell 160a and the active cell 160b experience the same fabrication steps in their respective vicinities (e.g., the same distances away). As a result, the active cells 160a, 160b respond uniformly to external pressure of the fluid within the lumen 104 of the anatomy 102. The active cells 160a, 160b are also evenly spaced from the top edge 330 and the bottom edge 332 of the pressure sensor 150.

It is well known that lithography, deposition, and etching processes suffer from process non-uniformity and proximity effects. Accordingly, the exact surrounding of the active cells 160a, 160b matters. The thin membrane 168 is also sensitive to these process variabilities. The dummy cells 164a, 164b, 164c, 164d make the MEMS process as uniform as possible, so that the two active cells 160a, 160b behave the same within the pressure sensor 150, but also from die to die and even wafer to wafer. The dummy cells 164a, 164b, 164c, 164d are thus provided for uniformity. As shown in Figs. 10 and 11, the layout of the dummy cells 164a, 164b, 164c, 164d is symmetrical, e.g., the surroundings of dummy cells 164a, 164b, 164c, 164d is symmetrical along the axes 314, 316. As a result, any active cells 160a, 160b will see the same surrounding, and lithography, deposition and etching process will be as uniform as possible.

The active cells 160a, 160b are spaced from one another by a pitch 322. The pitch 322 can be any suitable value in various embodiments. In some embodiments, the pitch 322 can be between approximately 10 $\mu$m and approximately 40 $\mu$m, between approximately 15 $\mu$m and approximately 35 $\mu$m, and/or between approximately 22 $\mu$m and approximately 26 $\mu$m, including values such as 20 $\mu$m, 22 $\mu$m, 23 $\mu$m, 24 $\mu$m, 25 $\mu$m, 26 $\mu$m, 28 $\mu$m, and/or other suitable values both larger and smaller. The pitch 320 (between the dummy cell 164a, 164b and its nearest active cell 160a, 160b) is greater than the pitch 322 (between the active cells). In that regard, the spatially compact distribution of capacitive cells in the die 310 takes into account space for DRIE along the boundary 312. That is, the larger pitch 320 allows for the width of the DRIE lane along the boundary 312 (e.g., between the active cell 160b and the dummy cell 164b to the right of the active cell 160a). To preserve symmetry, the larger pitch 320 is also provided between the active cell 160a and the dummy cell 164a to the left of the active cell 160a. Accordingly, the dummy cells 164a, 164b, 164c, 164d can be positioned close to the active cells 160a, 160b to create a dense arrangement while still leaving space for the size and shape of the pressure sensors 150 to be defined and for the pressure sensors 150 be singulated along the boundary 312 with DRIE. The pitches 320, 322, 324 can be referenced as distances in some instances.

One or more of the dimensions described herein may be accurate within a tolerance of ± 10 $\mu$m, ± 5 $\mu$m, ± 3 $\mu$m, ± 2 $\mu$m, ± 1 $\mu$m, and/or other suitable values both larger and smaller. Tolerances of one or more dimensions described herein can be ± 3$\sigma$ in some instances.

Persons skilled in the art will also recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

**Claims**

1.  A capacitive pressure sensor for a pressure-sensing guidewire or catheter, the capacitive pressure sensor comprising:

    a substrate;
    a first active cell formed in the substrate;
    a second active cell formed in the substrate, wherein the first active cell and the second active cell are electrically active to generate electrical signals representative of an external pressure; and
    a dummy cell formed in the substrate, wherein the dummy cell is electrically inactive such that no electrical signal representative of the external pressure is generated.

2.  The capacitive pressure sensor of claim 1, wherein the first active cell and the second active cell are spaced from one another by a first pitch.

3.  The capacitive pressure sensor of claim 2, wherein the dummy cell is spaced from at least one of the first active cell or the second active cell by a different, second pitch.

4.  The capacitive pressure sensor of claim 1, wherein the first active cell, the second active cell, and the dummy cell are arranged longitudinally along the substrate.

5.  The capacitive pressure sensor of claim 1, wherein the first active cell and the second active cell comprise a first diameter, and wherein the dummy cell comprises a different, second diameter.

6.  The capacitive pressure sensor of claim 1, further comprising:

    an integrated circuit disposed in the substrate, wherein the integrated circuit is in communication with the first active cell and the second active cell.

7.  The capacitive pressure sensor of claim 6, further comprising:

    a first bond pad formed in the substrate; and
    a second bond pad formed in the substrate, wherein the first bond pad and the second bond pad are in communication with the integrated circuit.

8.  The capacitive pressure sensor of claim 1, wherein the first active cell and the second active cell are symmetrical about an axis of the substrate.

9.  The capacitive pressure sensor of claim 1, wherein the substrate comprises a proximal portion comprising a first dimension and a distal portion comprising a smaller, second dimension, and wherein the first active cell and second active cell are formed in the distal portion.

10. The capacitive pressure sensor of claim 9, further comprising:

    an integrated circuit disposed in the distal portion of the substrate, wherein the integrated circuit is in communication with the first active cell and the second active cell.

11. An intraluminal pressure-sensing device, comprising:

    a flexible elongate member configured to be positioned within a body lumen of a patient;
    a capacitive pressure sensor coupled to the flexible elongate member, wherein the capacitive pressure sensor comprises:

    a substrate;
    a first active cell formed in the substrate;
    a second active cell formed in the substrate, wherein the first active cell and the second active cell are electrically active to generate electrical signals representative of an external pressure; and
    a dummy cell formed in the substrate, wherein the dummy cell is electrically inactive such that no electrical signal representative of the external pressure is generated.

12. The intraluminal pressure-sensing device of claim 11, further comprising:

    an integrated circuit disposed in the substrate, wherein the integrated circuit is in communication with the first active cell and the second active cell, wherein the integrated circuit is configured to output an electrical signal representative of a sensed pressure at the first active cell and the second active cell.

13. The intraluminal pressure-sensing device of claim 12, further comprising:

    a first electrical conductor and a second electrical conductor only,
    wherein the first electrical conductor and the second electrical conductor are in communication with the integrated circuit,
    wherein the capacitive pressure sensor is coupled to a distal portion of the flexible elongate member,

wherein the first electrical conductor and the second electrical conductor extend from the distal portion of the flexible elongate member to a proximal portion of the flexible elongate member, and
wherein at least one of the first electrical conductor or the second electrical conductor are configured to transmit the electrical signal representative of the sensed pressure from the capacitive pressure sensor at the distal portion of the flexible elongate member to a connector at the proximal portion of the flexible elongate member.

14. The intraluminal pressure-sensing device of claim 13, further comprising:

a first bond pad formed in the substrate; and
a second bond pad formed in the substrate, wherein the first bond pad and the second bond pad are in communication with the integrated circuit, and
wherein the first electrical conductor and the second electrical conductor are respectively in communication with the first bond pad and the second bond pad.

15. The intraluminal pressure-sensing device of claim 11, further comprising:

a housing coupled to the flexible elongate member, wherein the capacitive pressure sensor is disposed within the housing.

16. The intraluminal pressure-sensing device of claim 15, wherein the substrate of the capacitive pressure sensor comprises a proximal portion coupled to the housing and a cantilevered distal portion, wherein the first active cell and the second active cell are formed in the cantilevered distal portion.

17. The intraluminal pressure-sensing device of claim 16, further comprising:

an integrated circuit disposed in the cantilevered distal portion of the substrate, wherein the integrated circuit is in communication with the first active cell and the second active cell.

18. The intraluminal pressure-sensing device of claim 11, wherein the flexible elongate member comprises a guidewire.

19. The intraluminal pressure-sensing device of claim 11, wherein the flexible elongate member comprises a catheter.

20. A system, comprising:

a pressure-sensing catheter or guidewire comprising a capacitive pressure sensor, wherein the capacitive pressure sensor comprises:

a substrate;
a first active cell formed in the substrate;
a second active cell formed in the substrate, wherein the first active cell and the second active cell are electrically active to generate electrical signals representative of an external pressure; and
a dummy cell formed in the substrate, wherein the dummy cell is electrically inactive such that no electrical signal representative of the external pressure is generated; and
a computer in communication with the capacitive pressure sensor, wherein the computer is configured to generate a pressure value based on the electrical signals representative of the external pressure and to output, to a display, a visual representation of the pressure value.

**Fig. 1**

**Fig. 2**

**Fig. 3**

**Fig. 4**

Fig. 5

**Fig. 6**

**Fig. 7**

Fig. 8

310

Fig. 10

**Fig. 9**

Fig. 10

**Fig. 11**

Europäisches
Patentamt
European
Patent Office
Office européen
des brevets

# EUROPEAN SEARCH REPORT

Application Number

EP 18 20 7789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/128702 A1 (DEGERTEKIN F LEVENT [US] ET AL) 23 May 2013 (2013-05-23) * paragraphs [0039] - [0043], [0077], [0078]; figures 2a,15 * | 1-20 | INV. A61B5/0215 |
| X | US 2007/118039 A1 (BODECKER VOLKER [DE] ET AL) 24 May 2007 (2007-05-24) * paragraphs [0070] - [0074], [0078], [0104], [0108], [0109]; figures 4,9-13 * | 1-20 | |
| A | US 2005/121734 A1 (DEGERTEKIN F L [US] ET AL) 9 June 2005 (2005-06-09) * paragraphs [0028] - [0034]; figures 1,2 * | 1-20 | |
| A | US 2016/000344 A1 (CAO ARIEL [US]) 7 January 2016 (2016-01-07) * paragraphs [0048] - [0050]; figures 2,3 * | 1-20 | |
| A | WO 2018/095991 A1 (KONINKLIJKE PHILIPS NV [NL]) 31 May 2018 (2018-05-31) * page 5, line 12 - page 7, line 28; figures 1,2 * | 1-20 | TECHNICAL FIELDS SEARCHED (IPC) A61B |

The present search report has been drawn up for all claims

| Place of search | Date of completion of the search | Examiner |
|---|---|---|
| The Hague | 20 May 2019 | Mecking, Nikolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 20 7789

This annex lists the patent family members relating to the patent documents cited in  the above-mentioned European search report.
The members are as contained in the European Patent Office EDP file on
The European Patent Office is in no way liable for these particulars which are merely given for the purpose of information.

20-05-2019

| Patent document cited in search report | | Publication date | Patent family member(s) | | Publication date |
|---|---|---|---|---|---|
| US 2013128702 | A1 | 23-05-2013 | NONE | | |
| US 2007118039 | A1 | 24-05-2007 | AU | 2006318295 A1 | 31-05-2007 |
| | | | CA | 2631057 A1 | 31-05-2007 |
| | | | EP | 1968433 A2 | 17-09-2008 |
| | | | JP | 2009517137 A | 30-04-2009 |
| | | | US | 2007118039 A1 | 24-05-2007 |
| | | | US | 2010174201 A1 | 08-07-2010 |
| | | | US | 2011201949 A1 | 18-08-2011 |
| | | | US | 2014081158 A1 | 20-03-2014 |
| | | | WO | 2007062299 A2 | 31-05-2007 |
| US 2005121734 | A1 | 09-06-2005 | US | 2005121734 A1 | 09-06-2005 |
| | | | WO | 2005046443 A2 | 26-05-2005 |
| US 2016000344 | A1 | 07-01-2016 | CN | 106714664 A | 24-05-2017 |
| | | | EP | 3164059 A1 | 10-05-2017 |
| | | | JP | 2017527329 A | 21-09-2017 |
| | | | US | 2016000344 A1 | 07-01-2016 |
| | | | WO | 2016004251 A1 | 07-01-2016 |
| WO 2018095991 | A1 | 31-05-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 18188185 A